# EUROPÄISCHE PATENTSCHRIFT

(11) **EP 0 933 095 B1**
(45) Veröffentlichungstag und Bekanntmachung des Hinweises auf die Patenterteilung: **26.11.2003**
(21) Anmeldenummer: 98250452.4
(22) Anmeldetag: 29.12.1998
(51) Int. Cl.: A61N 1/365

(54) **Selbstkalibrierender ratenadaptiver Herzschrittmacher**
Auto-calibrating rate adaptive pacemaker
Stimulateur cardiaque à fréquence adaptée et à autocalibration

(30) Priorität: 29.01.1998 DE 19804843
(43) Veröffentlichungstag der Anmeldung: 04.08.1999
(73) Patentinhaber: BIOTRONIK Mess- und Therapiegeräte GmbH & Co Ingenieurbüro Berlin, 12359 Berlin (DE)
(72) Erfinder: Meier, Jan H., Dr., 91080 Uttenreuth (DE)
(74) Vertreter: Christiansen, Henning, Dipl.-Ing.

(56) Entgegenhaltungen:
- EP-A- 0 325 851
- EP-A- 0 361 517
- EP-A- 0 654 285
- EP-A- 0 804 941
- US-A- 5 074 302
- US-A- 5 292 340
- US-A- 5 303 702

## Beschreibung

Die Erfindung betrifft einen selbstkalibrierenden ratenadaptiven Herzschrittmacher gemäß dem Oberbegriff des Anspruchs 1.

Ratenadaptive Herzschrittmacher, bei denen die Stimulationsfrequenz bzw. -rate in Abhängigkeit von im Körper des Patienten aufgenommenen Signalen eingestellt wird, die den physiologischen Bedarf des Patienten in Bezug auf die Herzaktivität reflektieren, sind seit Jahren in vielgestaltigen Ausführungen bekannt und im klinischen Einsatz. Einen Überblick über die mit der Entwicklung der Ratenadaption in der Schrittmachertechnik verfolgten Ziele und die eingeschlagenen Wege gibt K. Stangl et al.: Frequenzadaptive Herzschrittmacher, Darmstadt 1990.

Es ist auch eine Vielzahl von Anordnungen zur Impedanzmessung im Bereich des Thorax oder im Herzen zur Gewinnung eines Impedanzsignals für ratanadaptive Herzschrittmacher bekannt, so daß die Technik der intrakardialen Impedanzmesung als solche dem Fachmann vertraut ist. Die meisten dieser Anordnungen sind dazu ausgelegt, ein das Atem- bzw. Minutenvolumen als Ausdruck der körperlichen Belastung des Patienten und als eigentlichem Ratensteuerparameter repräsentierendes Signal zu gewinnen; vgl. dazu beispielsweise EP 0 151 689 B1, EP 0 249 818 oder DE 42 31 601 A1.

Bekannt ist auch das sogenannte ResQ-Verfahren (Regional Effective Slope Quality) (SCHALDACH, Max: Electrotherapy of the Heart, 1.Aufl., Springer-Verlag, S.114ff.), bei dem der zeitliche Verlauf der intrakardialen Impedanz zur Bestimmung der physiologisch sinnvollen adaptiven Herzrate herangezogen wird.

Diesem Verfahren liegt die Erkenntnis zugrunde, daß die intrakardiale Impedanz in einem bestimmten Zeitfenster nach einem QRS-Komplex - der sogenannten "region of interest" (ROI) - eine besonders signifikante Abhängigkeit von der Belastung des Organismus aufweist.

Es wird deshalb die Steigung der Impedanzkurve in der ROI bestimmt und die Differenz zwischen der Steigung einer Ruhe- bzw. Referenzkurve und der Steigung der aktuell gemessenen Impedanzkurve (Belastungskurve) berechnet. In Abhängigkeit von dieser Differenz wird die adaptive Herzrate eingestellt. Die Zuordnung der berechneten Steigungsdifferenz zu der einzustellenden Herzrate erfolgt auch hier durch eine Kennlinie. Da diese für verschiedene Menschen unterschiedlich und vom körperlichen Zustand abhängig ist, muß der Herzschrittmacher für jeden Träger individuell kalibriert werden, und die Kalibrierung ist bei wesentlich verändertem Gesundheitszustand und Belastungsvermögen oder veränderten Lebensumständen des Patienten zu wiederholen, wobei auch die Lage der ROI zu überprüfen ist.

Verschiedene Vorschläge zur Selbstadaption (Autokalibration) ratenadaptiver Herzschrittmacher finden sich in US 5 292 340 A, EP 0 325 851 A2, US 5 074 302 oder EP 0 654 285 A2. In US 5 303 702 wird für die Selbstadaption eines aufgrund des Minutenvolumens gesteuerten Schrittmachers eine Trendberechnung vorgeschlagen.

In WO 93/20889 wird eine Zwei-Sensoren-Anordnung mit einer Schaltung zur Erfassung des Minutenvolumens und einem zusätzlichen Aktivitätsfühler vorgeschlagen, bei der die Stimulationsrate in Abhängigkeit von den für die einzelnen Fühler ableitbaren Zielraten bestimmt wird.

In der europäischen Patentanmeldung EP 97 250 057.3 der Anmelderin wird ein impedanzgesteuerter Schrittmacher vorgeschlagen, der ohne patientenspezifische Kalibrierung auskommen kann und sich an veränderte Randbedingungen selbsttätig anpaßt. Bei diesem Schrittmacher wird das Zeitintegral der Impedanz über einen vorbestimmten Abschnitt des Herzzyklus als primäre Impedanzgröße bestimmt und zur Ratenadaption genutzt.

Es ist Aufgabe der Erfindung, einen zuverlässig und mit akzeptablem Rechenaufwand und Stromverbrauch selbstkalibrierend arbeitenden Herzschrittmacher der gattungsgemäßen Art anzugeben.

Diese Aufgabe wird durch einen Herzschrittmacher mit den in Anspruch 1 angegebenen Merkmalen gelöst.

Die Erfindung schließt den Gedanken ein, den zeitlichen Verlauf der relevanten physiologischen Größe - speziell der intrakardialen Impedanz - während einer Kalibrationsphase zur Ableitung einer Schar von Modell-Stimulationsparameterkurven zu nutzen, aus der durch Vergleich mit einer unabhängig von der Erfassung der besagten physiologischen Größe (Impedanz) gewonnenen Stimulationsparameterkurve die geeignetste für die weitere Schrittmachersteuerung ausgewählt wird. Das wird in der Regel diejenige auf der beasgten Größe basierte Kurve sein, deren Verlauf am stärksten mit dem der unabhängig von dieser Größe ermittelten Kurve korreliert.

Die Auswertung von Impedanzmessungen erfolgt gemäß dem oben skizzierten ResQ- bzw. Steigungsverfahren derart, daß zu jedem Zeitpunkt n Stimulationsratenwerte auf der Basis von n verschiedenen Paaren von Abtastzeitpunkten an der Impedanzkurve berechnet werden. Die Zeitabhängigkeit der zu einem festen Abtastzeitpunkt-Paar gehörenden Ratenwerte bildet jeweils eine zur Auswahl stehende Modell-Stimulationsratenkurve.

Der erfindungsgemäße Herzschrittmacher wertet in einer bevorzugten Ausführung die intrakardiale Impedanz, insbesondere die unipolar gemessene rechtsventrikuläre Impedanz, in einem breiten Bereich aus, der die üblicherweise für einzelne Patienten eingestellten ROI-Bereiche einschließt. Auch die Abtastzeitpunkt-Paare sind so vorprogrammiert, daß sie mit Sicherheit das für den betreffenden Patienten "optimale" Paar enthalten. Ihre Festlegung bedarf jedoch keiner patientenindividuellen Programmierung nach der Implantation, sondern sie werden bevorzugt bei der Herstellung des Schrittmachers in einem Festwertspeicher gespeichert.

Die die Abhängigkeit der Stimulationsrate von der Impedanzgröße bestimmende Kennlinie als wesentlicher Betriebsparameter der Ratenbestimmungseinrichtung ist nicht notwendigerweise statisch, sondern kann kontinuierlich oder in bestimmten Zeitabständen optimiert werden, um insbesondere eine Anpassung der Variationsbreite der Impedanzgröße an die zulässige Variationsbreite der Herzrate zu erreichen.

Die Variationsbreite ist bei dieser Ausführung zu Beginn des Betriebs nicht bekannt, sondern wird durch laufende Messung der Impedanz während des Betriebs ermittelt und nach jeder Messung optimiert. Zu Beginn des Betriebs wird ein Schätzwert für den unteren und den oberen Grenzwert der Variationsbreite als Startwert vorgegeben.

Bei der Einstellung sind dann zwei Fälle zu unterscheiden: Erstens kann ein Meßwert der Impedanz die bisher ermittelte Variationsbreite nach oben oder unten überschreiten. In diesem Fall wird die Variationsbreite entsprechend erweitert und dadurch aktualisiert. Die Zeitkonstante dieses Anpassungsvorgangs liegt vorzugsweise in der Größenordnung weniger Sekunden, um eine schnelle Anpassung zu erreichen und eine - auch nur kurzzeitig - überhöhte Herzrate zu verhindern. Zweitens kann der Fall auftreten, daß die Impedanz die zuvor bestimmte Variationsbreite über einen längeren Zeitraum nicht mehr voll ausschöpft. In diesem Fall kann die Variationsbreite langsam (mit einer Zeitkonstante vorzugsweise in der Größenordnung von Wochen) wieder verringert werden. Die Stmulationsrate-Impedanz-Kennlinie weist dem unteren Grenzwert der Variationsbreite der Aktivitätsgröße die Basisrate zu und entsprechend dem oberen Grenzwert der Aktivitätsgröße die maximale Stimulationsrate. Durch eine Veränderung dieser Grenzwerte während der Optimierung ändert sich folglich auch die Kennlinie selbst.

Vorteilhafte Weiterbildungen der Erfindung sind weiterhin in den Unteransprüchen gekennzeichnet bzw. werden nachstehend zusammen mit der Beschreibung einer bevorzugten Ausführung der Erfindung anhand der Figuren näher dargestellt.

Es zeigen:
- Figur 1: ein Funktions-Blockschaltbild eines Herzschrittmachers als Ausführungsbeispiel der Erfindung,
- Figur 2: jeweils einen Abschnitt zweier intrakardial aufgenommener Leitfähigkeits-Meßkurven zur Illustration der Gewinnung der Impedanzgröße RQ bei einer bevorzugten Ausführung der Erfindung und
- Figur 3: in einer Vergleichsdarstellung den Zeitverluuf einer aufgrund von Aktivitätssignalen berechneten Referenz-Stimulationsrate und zweier aufgrund von Impedanzsignalen bei unterschiedlicher Einstellung der RQ-Berechnungspunkte gemäß Fig. 2 berechneter Auswahl-Stimulationsraten.

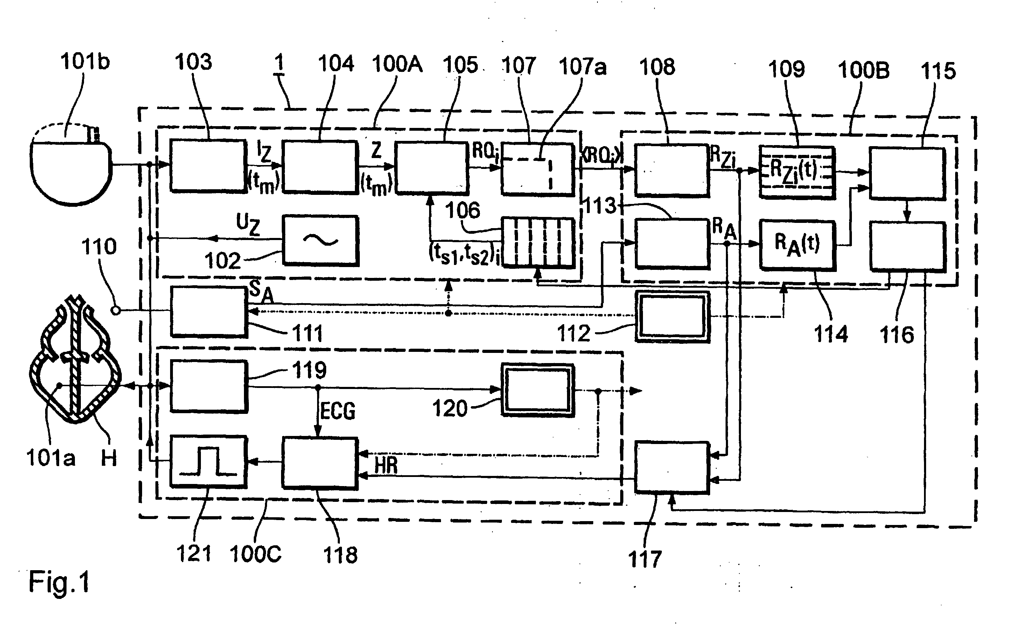

Figur 1 zeigt als Ausführungsbeispiel der Erfindung einen ratenadaptiven Herzschrittmacher 100 als fragmentarisches Funktions-Blockschaltbild, in dem nur die für die Erläuterung der Erfindung wichtigen Komponenten dargestellt sind. Der übrige Aufbau entspricht dem bekannter Herzschrittmacher.

An den Herzschrittmacher 100 ist eine unipolare Schrittmacher- und Meßelektrode 101a im rechten Ventrikel des Herzens H zur Herzsignalerfassung, zur Massung der rechtsventrikulären intrakardialen Impedanz Z und als Reizelektzrode zugeordnet. Als Gegenelektrode dient eine Gehäuseelektrode 101b.

Die Impedanzmessung erfolgt in an sich bekannter Weise getaktet, indem die Elektrode 101a über einen Meßspannungsgenerator 102 mit einer Meßspannung beaufschlagt und über eine Strommeßschaltung 103 zu m äquidistant vorprogrammierten Zeitpunkten tₘ der Strom I_{Z}(tₘ) zwischen der Meßelektrode 101a und der Gegenelektrode 101b gemessen wird. Die den zeitlichen Verlauf der Impedanz kennzeichnenden Werte Z(tₘ) ergeben sich am Ausgang einer der Strommeßschaltung 103 nachgeschalteten Impedanzberechnungsstufe 104 als Quotienten aus der (fest voreingestellten) Meßspannung und den gemessenen Stromwerten.

In einer RQ-Berechnungsstufe 105, die über einen Steuereingang mit einem Abtastzeitspeicher 106 verbunden ist, wird für jeden erfaßten Impedanz-Zeitverlauf Z(tₘ) eine Auswahlmenge von n Ratensteuerparameterwerten RQᵢ berechnet, indem die Differenz (Z(tₛ₁) - Z(tₛ₂)) der Impedanzwerte für jedes von n Abtastzeit-Wertepaaren (tₛ₁, tₛ₂)ᵢ durch den zugehörige Zeitabstand (tₛ₁ - tₛ₂)ᵢ dividiert wird.

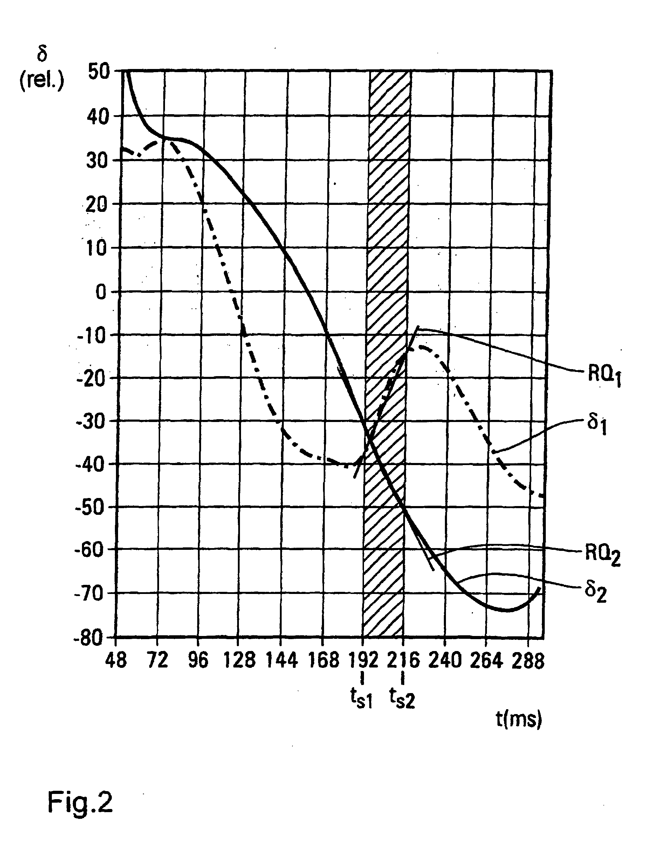

In Fig. 2 ist die an einer unipolaren Elektrode gemessene Leitfähigkeit σ (in relativen Einheiten) für zwei unterschiedliche Belastungszustände eines Patienten (durchgezogene Linie = hohe Belastung, strichpunktierte Linie = Ruhe) über der Zeit nach einem Anregungsimpuls aufgetragen. Im Bereich zwischen den Zeitpunkten tₛ₁, tₛ₂ unterscheidet sich der Kurvenverlauf am stärksten, so daß die Anstiege RQ₁, RQ₂ der Kurven in diesem Zeitbereich einne brauchbaren Ratensteuerparameter ergeben können. Der Anstieg wird durch den Quotienten aus der Differenz der Leitfähigkeits- bzw. der hierzu reziproken Impedanz-Meßwerte zu den Abtastzeitpunkten und deren Abstand approximiert.

In einer der RQ-Berechnungsstufe nachgeschalteten Nachbearbeitungsstufe 107 werden mit Hilfe eines (als solchen bekannten) Glättungsalgorithmus atmungsbedingte sbrupte Schlag-zu-Schlag-Änderungen der Ratensteuerparameterwerte infolge entsprechender Impedanzschwankungen ausgefiltert, und am Ausgang der Stufe 107 steht eine Menge geglätteter RQ-Werte <RQᵢ> zur Verfügung. Die Nachbearbeitungsstufe 107 weist zur Ausführung der Nachbearbeitung insbesondere einen internen FIFO-Arbeitsspeicher 107a auf, in dem jeweils gleitend für eine vorbestimmte Folge aufeinanderfolgender Herzschläge die zugehörigen RQi-Werte zur Ausführung der Nachbearbeitung gespeichert werden. Für die Stufe 107 sind wahlweise weitere Funktionen programmierbar, so etwa ein Algorithmus zur Verringerung des Einflusses des sogenannten Orthostaseeffektes, d.h. zur teilweisen Eliminierung irregulärer Impedanzschwankungen, die lediglich durch Lageänderungen des Patienten bedingt und ohne Signifikanz für die Ratensteuerung sind.

Die oben erläuterten Komponenten 102 bis 107 bilden eine Impedanzverarbeitungseinrichtung 100A des Schrittmachers 100.

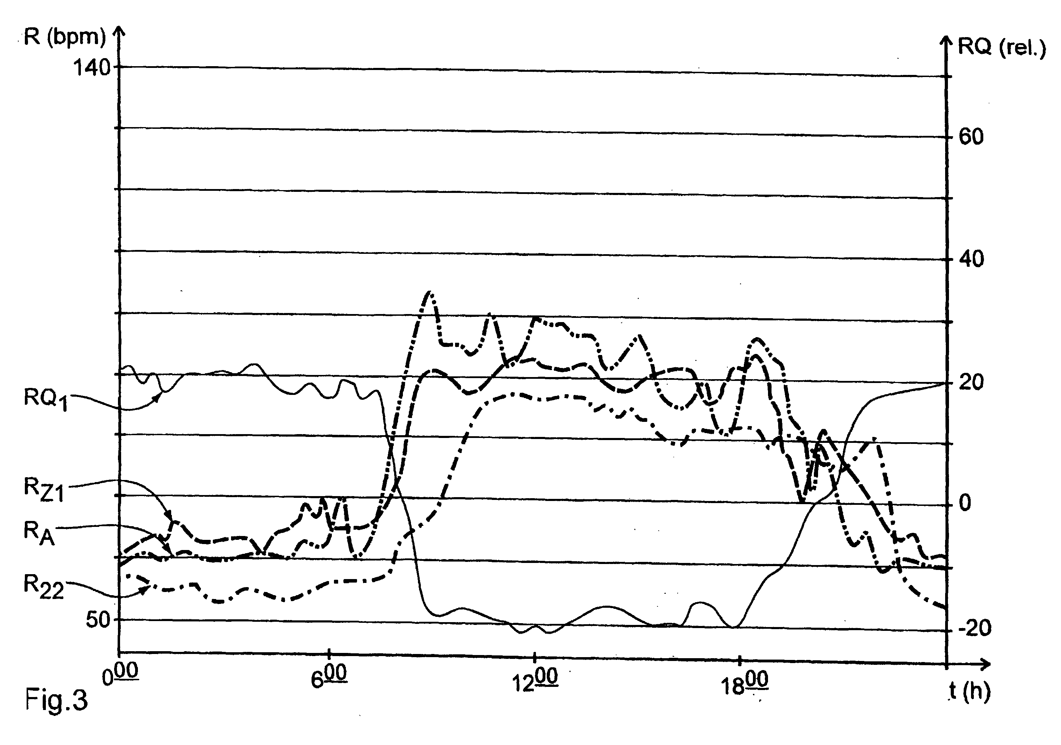

Der Nachbearbeitungsstufe 107 nachgeschaltet ist eine Ratenberechnungseinrichtung 108, in der auf der Basis jedes der geglätteten Werte der Ratensteuerparameter <RQᵢ> nach einem fest programmierten Algorithmus jeweils ein impedanzbasierter Wert R_{Zi} der Stimulationsrate berechnet wird. Mit dem Ausgang der Ratenberechnungseinrichtung 108 ist ein Raten-Zeitverlaufsspeicher 109 mit n Speicherbereichen verbunden, in dem die Zeitabhängigkeiten der einzelnen impedanzbasiert unter Zugrundelegung verschiedener Abtastzeitpunkte berechneten Ratenwerte R_{Zi}(t) über einen vorbestimmten Kalibrationszeitraum (vgl. dazu Fig. 3) gespeichert werden.

Dem Schrittmacher 100 ist ein Aktivitätssensor 110 zugeordnet, dessen Signale in einer Aktivitätssignal-Vorverarbeitungsstufe 111 in an sich bekannter Weise einer Verstarkung und Filterung unterzogen werden.

Eine Ratenbestimmungs-Zeitsteuerung 112 steuert sowohl die oben skizzierte Impedanzerfassung und -auswertung in der Stufe 100A als auch die Aktivitätssignalerfassung und -auswertung. Die Impedanzmessungen und Aktivitäterfassungen werden durch die Ablaufsteuerung 112 zu in einem internen Zeitprogrammspeicher vorprogrammierten Zeitpunkten innerhalb eines 24h-Kalibrationszeitraumes und erstere zudem in Synchronität zu stimulierten oder spontanen Herzereignissen ausgelöst. Auch die Wiederholungsperiode der Kalibrationszyklen ist in der Zeitsteuerung 112 intern programmiert.

Das vorverarbeitete Aktivitätssignal S_{A} wird einer Referenzraten-Berechnungsstufe 113 zugeführt, wo nach einem an sich bekannten Algorithmus ein aktivitätsbasierter Wert R_{A} der Stimulationsrate berechnet wird. In einem der Stufe 113 nachgeschalteten Referenzraten-Zeitverlaufsspeicher 114 mit ebenfalls n Speicherbereichen wird die Zeitabhängigkeit der aktivitätsbasiert berechneten Ratenwerte R_{A}(t) über den Kalibrationszeitraum (vgl. dazu wiederum Fig. 3) gespeichert.

Der Raten-Zeitverlaufsspeicher 109 und der Referenzraten-Zeitverlaufsspeicher 114 sind mit den beiden Signaleingängen einer Korrelatorstufe 115 verbunden, in der die n Korrelationskoeffizienten der einzelnen impedanzbasierten Raten-Zeitabhängigkeiten R_{Zi}(t) bezüglich des Referenzraten-Zeitverlaufes R_{A}(t) ermittelt werden. Der Ausgang der Korrelatorstufe 115 ist mit einer Entscheidungseinrichtung 116 verbunden, in der der Maximalwert des Korrelationskoeffizienten bestimmt und dasjenige Abtastzeit-Wertepaar (tₛ₁, tₛ₂)ⱼ als das für die impedanzbasierte Ratenberechnung optimale definiert wird, für das die Zeitabhängigkeit der Rate am stärksten mit der Zeitabhängigkeit der aufgrund des Aktivitätssignals errechneten Rate korreliert.

In Fig. 3 sind beispielartig zwei Zeitverläufe von auf Impedanzbasis errechneten Stimulationsraten R_{Z1}, R_{Z2} dem Zeitverlauf der auf Aktivitätsbasis errechnten Rate R_{A} gegenübergestellt. Zudem ist in der Figur (als dünn durchgezogene Kurve) ein Beispiel für den zeitlichen Verlauf eines Ratensteuerparameters RQ angegeben; diese Zeitabhängigkeit wird aber als solche in der hier beschriebenen Anordnung nicht zusammenhängend ermittelt. Von den beiden gezeigten impedanzbasierten Kurven hat die für R_{Z1} offensichtlich den höheren Korrelationskoeffizienten mit R_{A}.

Die Stufen 108, 109 und 113 bis 116 bilden zusammen die Ratenbestimmungseinrichtung 100B des Schrittmachers.

Die Entscheidungseinrichtung 116 gibt ein entsprechendes Steuersignal an die Speicherzugriffssteuerung des Abtastzeitspeichers 106 aus, aufgrund dessen für den nachfolgenden Schrittmacherbetrieb (bis zur nächsten Kalibrationsprozedur) ausschließlich dieses j-te Wertepaar der Ermittlung des Ratensteuerparameters RQⱼ zugrundegelegt wird. Zudem wird ein Steuersignal an eine Ratensteuer-Umschalteinheit 117 ausgegeben, an deren zwei Signaleingängen das Ausgangssignal R_{Zj} der (impedanzbasierten) Ratenberechnungseinrichtung 108 und dasjenige der (aktivitätsbasierten) Referenzratenberechnungseinrichtung 113 anliegen. In der Ratensteuer-Umschalteinheit 117 wird nach Vorliegen des Kalibrationsergebnisses von der während der Kalibrationsphase eingestellten aktivitätsbasierten Ratensteuerung des aktuellen Schrittmacherbetriebs auf die impedanzbasierte Ratensteuerung umgeschaltet.

Der am Ausgang der Umschalteinheit 117 anliegende aktuelle Ratenwert HR wird einem Stimulationsimpulsgenerator 118 zugeführt, der zusammen mit einer EKG-Eingangsstufe 119, einer Schrittmacherbetriebssteuerung 120 und einer Ausgangsstufe 121 sowie weiteren an sich bekannten Komponenten den eigentlichen Schrittmacherblock 100C bildet. Die Funktion dieser Komponenten wird hier als bekannt vorausgesetzt.

Zu beachten ist, daß die in Fig. 1 gezeigte Signalverbindung der Schrittmachersteuerung 120 zur Meßelektrode 101a, mit der auch die Herzaktionen bzw. intrakardialen EKGs erfaßt werden, eine Unterscheidung zwischen spontanen und evozierten Herzaktionen und damit eine berücksichtigung des Ereignistyps bei der impedanzbasierten Reatenberechnung ermöglicht. Insbesondere kann der errechneten Stimulationsrate bei jedem Ereignistypwechsel ein Ratenoffset hinzugefügt werden, dessen Betrag in Abhängigkeit vom vorhergehenden und vom aktuellen Ratenwert so gewählt ist, daß der Ratensprung einen vorbestimmten Betrag nicht überschreitet, und der bei den nachfolgenden Herzereignissen stufenweise bis auf Null zurückgeführt wird. Die konkreten schaltungstechnischen Mittel zur Realisierung dieser Zusatzfunktion stehen dem Fachmann aus bekannten Anordnungen zur Ratenglättung bzw. -angleichung zur Verfügung.

Die Erfindung beschränkt sich in ihrer Ausführung nicht auf die vorstehend angegebenen bevorzugten Ausführungsbeispiele. Vielmehr ist eine Anzahl von Varianten denkbar, welche von der beanspruchten Lösung auch in anders gearteter Ausführung Gebrauch machen.

So kann anstelle des Aktivitätssensors auch ein anderer Fühler zur Kalibration dienen, der eine die Belastung des Patienten repräsentierende physiologische Größe liefert, Zur verbesserten Berücksichtigung des Orthostaseeffekts kann zusätzlich ein Positionsfühler vorgesehen sein, dessen Signale über einen geeigneten Algorithmus die Verarbeitung des Impedanzsignals beeinflussen. Alternativ zur letztgenannten Weiterbildung kann bei der Korrelationsbestimmung eine niedrigere Gewichtung der in Ruhephasen aufgenommenen Impedanzwerte gegenüber den Kurvenanteilen in Belastungsphasen vorgenommen werden, denn in Ruhephasen ist der Orthostaseeffekt am störendsten.

Bei der Kalibration ist nicht unbedingt ein vorgegebener Zeitraum einzuhalten, sondern sie kann auch bei Erreichung eines vorgegebenen Schwellwertes der Korrelationsgröße beendet werden. Diese Variante kann neben einer gewissen Stromersparnis insbesondere den Vorteil erbringen, daß der normale Schrittmacherbetrieb früher wieder aufgenommen werden kann. Zu ihrer Realisierung sind entsprechende Mittel zur Speicherung des Korrelationsgrößen-Schwellwertes und zur Ausführung eines wiederholten Vergleiches der im aktuellen Kalibrationsstatus errechneten Korrelationsgrößenwerte mit diesem gespeicherten Schwellwert vorzusehen.

## Patentansprüche

1. Selbstkalibrierender ratenadaptiver Herzschrittmacher (100), mit
einer Meß- und Verarbeitungseinrichtung (100A) zur Messung des zeitlichen Verlaufes einer physiologischen Größe (Z(tₘ)) über einen vorbestimmten Abschnitt eines Herzzyklus und zur Gewinnung eines Verlaufsparameters aus dem zeitlichen Verlauf,
einer der Meß- und Verarbeitungseinrichtung nachgeschalteten und durch eine Ablaufsteuerung (112) gesteuerten Stimulationsparameter-Bestimmungseinrichtung (100B, 117) zur Bestimmung eines Stimulationsparameterwertes, insbesondere der adaptiven Stimulationsrate (HR),
einem mit einem Eingang der Stimulationsparameter-Bestimmungseinrichtung verbundenen Körperfühler (110) zur Erfassung einer Belastungsgröße, dessen Signal zur Bestimmung des Stimulationsparameterwertes (HR) unter Nutzung des Verlaufsparameters mit herangezogen wird und
einem Stimulatoreinheit (100C) zur Erzeugung und Ausgabe von Stimulationsimpulsen mit dem bestimmten Stimulationsparameterwert,
**dadurch gekennzeichnet, daß**
die Meß- und Verarbeitungseinrichtung zur Gewinnung einer Mehrzahl verschiedener Werte (RQᵢ) des Verlaufsparameters in Abhängigkeit von mindestens einer Einstellgröße (tₛ₁, tₛ₂) in einer Kalibrationsphase ausgebildet ist und
die Stimulationsparameter-Bestimmungseinrichtung
eine der Meß- und Verarbeitungseinrichtung nachgeschaltete Stimulationsparameter-Berechnungseinrichtung (108) zur Berechnung einer Mehrzahl von Stimulationsparameter-Auswahlwerten (R_{Zi}) in Abhängigkeit von jeweils einem Wert des Verlaufsparameters,
eine dem Körperfühler nachgeschaltete Referenzparameter-Berechnungseinrichtung (113) zur Berechnung eines aktivitätsbestimmten Referenzparameterwertes (R_{A}),
eine eingangsseitig mit der Stimulationsparameter-Berechnungseinrichtung und der Referenzparameter-Berechnungseinrichtung verbundene Vergleichereinheit (115) zum Vergleich der Parameterauswahlwerte mit dem Referenzparameterwert und
eine eingangsseitig mit dem Ausgang der Vergleichereinheit verbundene Entscheidungseinrichtung (116) zur Auswahl eines der den Paremeterauswahlwerten zugeordneten Einstellgrößenwerte der Meß- und Verarbeitungseinrichtung als aktueller Einstellgrößenwert für die weitere Schrittmachersteuerung im wesentlichen aufgrund des Verlaufsparameters im Ergebnis des Vergleiches aufweist.

2. Herzschrittmacher nach Anspruch 1, **dadurch gekennzeichnet, daß** die Meß- und Verarbeitungseinrichtung zur Messung und Verarbeitung der intrakardialen Impedanz als physiologische Größe ausgebildet ist.

3. Herzschrittmacher nach Anspruch 1 oder 2, **dadurch gekennzeichnet, daß**
die Ablaufsteuerung (112) zur gleichzeitigen Aufnahme der Zeitabhängigkeit der Mehrzahl von Parameterauswahlwerten und des Referenzparameterwertes während eines Kalibrationszeitraumes ausgebildet ist,
die Parameterberechnungseinrichtung (108) und die Referenzparameter-Berechnungseinrichtung (113) zur Aufnahme der Zeitabhängigkeit ausgebildet und ihnen Speicher (109, 114) zur Speicherung der Zeitabhängigkeit zugeordnet sind und die Vergleichereinheit (115) als Korrelationsberechnungseinheit zur Bestimmung der Werte einer Korrelationsgröße für die Zeitabhängigkeit der einzelnen Paremeterauswahlwerte (R_{Zi}(t)) und diejenige des Referenzparemeterwertes (R_{A}(t)) und zur Ausgabe der Korrelationsgrößenwerte an die Entscheidungseinrichtung (116) ausgebildet ist.

4. Herzschrittmacher nach Anspruch 2 oder 3, **dadurch gekennzeichnet, daß** die Impedanzmeß- und -verarbeitungseinrichtung (105) zur Bestimmung des Anstieges der Impedanz-Zeit-Kurve in einem durch ein Wertepaar (tₛ₁, tₛ₂) aus einem Anfangs- und einem Endzeitpunkt nach einem Anregungsimpuls als Einstellgröße bestimmten Abtastzeitintervall ausgebildet ist.

5. Herzschrittmacher nach Anspruch 3 oder 4, **dadurch gekennzeichnet, daß** die Stimulationsparameter-Bestimmungseinrichtung zur schrittweise iterativen Berechnung der Korrelationsgröße und des Referenzparameterwertes ausgebildet ist.

6. Herzschrittmacher nach Anspruch 4, **dadurch gekennzeichnet, daß** die Impedanzmeß- und -verarbeitungseinrichtung (100A) einen Speicher (106) zur Speicherung einer vorbestimmten Menge von Wertepaaren aus Anfangs- und Endzeitpunkten aufweist.

7. Herzschrittmacher nach einem der vorhergehenden Ansprüche, **dadurch gekennzeichnet, daß** die Meß- und Verarbeitungseinrichtung (100A) Mittel (107) zur Glättung von Kurzzeitschwankungen und/oder von lageänderungsbedingten Schwankungen der Verlaufsparemeterwerte aufweist.

8. Herzschrittmacher nach einem der vorhergehenden Ansprüche, **dadurch gekennzeichnet, daß** der Körperfühler ein Aktivitätssensor (110) ist.

9. Herzschrittmacher nach einem der vorhergehenden Ansprüche, **dadurch gekennzeichnet, daß** der Stimulationsparameter-Bestimmungseinrichtung (100B) eine Stimulationssteuer-Umschalteinheit (117) zur wahlweisen Umschaltung auf körperfühlergesteuerten Schrittmacherbetrieb zugeordnet ist.

## Claims

1. A self-calibrating rate-adaptive cardiac pacemaker (100) comprising
a measuring and processing device (100A) for measuring the variation in time of a physiological variable (Z(tₘ)) over a predetermined portion of a heart cycle and for obtaining a variation parameter from the variation in time,
a stimulation parameter determining device (100B, 117) connected downstream of the measuring and processing device and controlled by an operating procedure control means (112), for determining a stimulation parameter value, in particular the adaptive stimulation rate (HR),
a body sensor (110) connected to an input of the stimulation parameter determining device for detecting an exertion variable, the signal of which is used for determining the stimulation parameter value (HR) while also using the variation parameter, and
a stimulator unit (100C) for generating and outputting stimulation pulses at the determined stimulation parameter value,
**characterised in that**
the measuring and processing device is adapted to obtain a plurality of different values (RQᵢ) of the variation parameter in dependence on at least one setting parameter (tₛ₁, tₛ₂) in a calibration phase, and
the stimulation parameter determining device comprises
a stimulation parameter calculating device (108) connected downstream of the measuring and processing device for calculating a plurality of stimulation parameter selection values (R_{Zi}) in dependence on a respective value of the variation parameter,
a reference parameter calculating device (113) connected downstream of the body sensor for calculating an activity-determined reference parameter value (R_{A}),
a comparator unit (115) connected at the input side to the stimulation parameter calculating device and the reference parameter calculating device for comparing the parameter selection values to the reference parameter value, and
a decision device (116) connected at the input side to the output of the comparator unit for selecting one of the setting parameter values, associated with the parameter selection values, of the measuring and processing device as the current setting parameter value for further pacemaker control substantially on the basis of the variation parameter as the result of the comparison.

2. A cardiac pacemaker according to claim 1 **characterised in that** the measuring and processing device is adapted to measure and process the intracardial impedance as the physiological variable.

3. A cardiac pacemaker according to claim 1 or claim 2 **characterised in that**
the operating procedure control means (112) is adapted for simultaneously recording the time dependency of the plurality of parameter selection values and the reference parameter value during a calibration period,
the parameter calculating device (108) and the reference parameter calculating device (113) are adapted to record the time dependency and associated therewith are memories (109, 114) for storing the time dependency, and
the comparator unit (115) is in the form of a correlation calculating unit for determining the values of a correlation variable for the time dependency of the individual parameter selection values (R_{Zi}(t)) and that of the reference parameter value (R_{A}(t)) and for outputting the correlation variable values to the decision device (116).

4. A cardiac pacemaker according to claim 2 or claim 3 **characterised in that** the impedance measuring and processing device (105) is adapted to determine the rise in the impedance-time curve in a scanning time interval which is determined by a pair of values (tₛ₁, tₛ₂) from a beginning and an end time after a stimulation pulse as a setting parameter.

5. A cardiac pacemaker according to claim 3 or claim 4 **characterised in that** the stimulation parameter determining device is adapted for stepwise iterative calculation of the correlation variable and the reference parameter value.

6. A cardiac pacemaker according to claim 4 **characterised in that** the impedance measuring and processing device (100A) has a memory (106) for storing a predetermined quantity of pairs of values from starting and end times.

7. A cardiac pacemaker according to one of the preceding claims **characterised in that** the measuring and processing device (100A) has means for smoothing short-term fluctuations and/or fluctuations governed by changes in position in the variation parameter values.

8. A cardiac pacemaker according to one of the preceding claims **characterised in that** the body sensor is an activity sensor (110).

9. A cardiac pacemaker according to one of the preceding claims **characterised in that** associated with the stimulation parameter determining device (100B) is a stimulation control change-over switching unit (117) for selectively switching over to body sensor-controlled pacemaker operation.

## Revendications

1. Stimulateur cardiaque (100) à autocalibrage et à fréquence adaptative, comportant
un dispositif de mesure et de traitement (100A) pour mesurer la variation dans le temps d'une grandeur physiologique (Z(tₘ)) pendant une section prédéterminée d'un cycle cardiaque et pour l'obtention d'un paramètre de variation à partir de la variation dans le temps,
un dispositif (100B, 117) de détermination de paramètres de stimulation, branché en aval du dispositif de mesure et de traitement et commandé par une unité de commande d'exécution (112) et servant à déterminer un paramètre de stimulation, notamment la fréquence adaptative de stimulation (HR),
un capteur corporel (110), relié à une entrée du dispositif de détermination du paramètre de stimulation et servant à détecter une grandeur de sollicitation, dont le signal est utilisé conjointement pour déterminer la valeur du paramètre de stimulation (HR) moyennant l'utilisation du paramètre de variation, et
une unité de stimulateur (100C) pour produire et délivrer des impulsions de stimulation avec la valeur déterminée du paramètre de stimulation,
**caractérisé en ce que**
le dispositif de mesure et de traitement est conçu pour obtenir une multiplicité de différentes valeurs (RQᵢ) du paramètre de variation en fonction d'au moins une valeur de réglage (tₛ₁, tₛ₂) dans une phase de calibrage, et
le dispositif de détermination du paramètre de stimulation comporte
un dispositif (108) de calcul du paramètre de stimulation, branché en aval du dispositif de mesure et de traitement et servant à calculer une multiplicité de valeurs (R_{Zi}) de sélection du paramètre de stimulation en fonction d'une valeur respective du paramètre de variation,
un dispositif (113) de calcul d'un paramètre de référence, branché en aval du capteur corporel et servant à calculer une valeur (R_{A}) du paramètre de référence, déterminée en fonction de l'activité,
une unité de comparaison (115) reliée côté entrée au dispositif de calcul du paramètre de stimulation et au dispositif de calcul du paramètre de référence et servant à comparer les valeurs de sélection de paramètre à la valeur de paramètre de référence, et
un dispositif de décision (116) relié côté entrée à la sortie de l'unité de comparaison et servant à sélectionner l'une des valeurs de grandeurs de réglage, associées aux valeurs de sélection de paramètre, du dispositif de mesure et de traitement en tant que valeur de grandeur de réglage actuelle pour la poursuite de la commande du stimulateur cardiaque essentiellement sur la base du paramètre de variation, en tant que résultat de la comparaison.

2. Stimulateur cardiaque selon la revendication 1, **caractérisé en ce que** le dispositif de mesure et de traitement est agencé de manière à mesurer et traiter l'impédance intracardiaque en tant que grandeur physiologique.

3. Stimulateur cardiaque selon la revendication 1 ou 2, **caractérisé en ce que**
l'unité de commande d'exécution (112) est conçue pour l'enregistrement simultané de la dépendance dans le temps de la pluralité de valeurs de sélection de paramètre et de la valeur de paramètre de référence pendant un intervalle de temps de calibrage,
le dispositif (108) de calcul du paramètre et le dispositif (113) de calcul du paramètre de référence sont conçus pour enregistrer la dépendance dans le temps, et des mémoires (109, 114) servant à mémoriser la dépendance dans le temps sont associées à ces dispositifs, et
l'unité de comparaison (115) est agencée sous la forme d'une unité de calcul de corrélation pour déterminer les valeurs d'une grandeur de corrélation pour la dépendance dans le temps des différentes valeurs (R_{Zi}(t)) de sélection de paramètre et pour déterminer la dépendance dans le temps de la valeur (R_{A}(t)) du paramètre de référence et pour délivrer les valeurs de grandeurs de corrélation au dispositif de décision (116).

4. Stimulateur cardiaque selon la revendication 2 ou 3, **caractérisé en ce que** le dispositif (105) de mesure et de traitement d'impédance est conçu pour déterminer la pente de la courbe de la variation de l'impédance dans le temps dans un intervalle de temps d'exploration déterminé par un couple de valeurs (tₛ₁, tₛ₂) formé par un instant initial et un instant final, après l'impulsion d'excitation en tant que grandeur de réglage.

5. Stimulateur cardiaque selon la revendication 3 ou 4, **caractérisé en ce que** le dispositif de détermination du paramètre de stimulation est conçu pour le calcul itératif pas-à-pas de la grandeur de corrélation et de la valeur du paramètre de référence.

6. Stimulateur cardiaque selon la revendication 4, **caractérisé en ce que** le dispositif de mesure et de traitement d'impédance (100A) comporte une mémoire (106) pour mémoriser une quantité prédéterminée de couples de valeurs à partir d'un instant initial et d'un instant final.

7. Stimulateur cardiaque selon l'une des revendications précédentes, **caractérisé en ce que** le dispositif de mesure et de traitement (100A) comporte des moyens (107) pour lisser des variations de brève durée et/ou des variations, conditionnées par une variation de longueur, des valeurs du paramètre de variation.

8. Stimulateur cardiaque selon l'une des revendications précédentes, **caractérisé en ce que** le capteur corporel est un capteur d'activité (110).

9. Stimulateur cardiaque selon l'une des revendications précédentes, **caractérisé en ce qu'**au dispositif (100A) de détermination des paramètres de stimulation est associée à une unité de commutation de commande de stimulation (117) pour réaliser une commutation au choix sur un fonctionnement de stimulateur commandé par un capteur corporel.
